# EUROPEAN PATENT SPECIFICATION

(11) **EP 2 099 435 B1**
(45) Date of publication and mention of the grant of the patent: **10.02.2010**
(21) Application number: 07862078.8
(22) Date of filing: 16.11.2007
(51) Int. Cl.: A61K 9/32, A61K 9/34, A61K 9/36

(54) **FILM-COATED SOLID DOSAGE FORM**
FESTE DOSIERFORM MIT FILMÜBERZUG
FORME GALÉNIQUE SOLIDE ENROBÉE D'UN FILM

(30) Priority: 30.11.2006 US 606463
(43) Date of publication of application: 16.09.2009
(73) Proprietor: Johnson & Johnson Consumer Companies, Inc., Skillman, NJ 08558 (US)
(72) Inventor: NELSON, Dennis, Memphis, TN 38117 (US); BELL, Anthony, Andover, New Jersey 07821 (US); SORG, Albert, Columbia, New Jersey 07832 (US)
(74) Representative: Kirsch, Susan Edith
(86) International application number: PCT/US2007/024078
(87) International publication number: WO 2008/066716

(56) References cited:
- WO-A-2004/075828
- US-A1- 2004 129 174

## Description

### FIELD OF THE INVENTION

The invention concerns film coated solid dosage forms. More particularly the invention concerns a taste releasing coated dosage form having a solid core, a first layer which is a "protective seal coat" and a second outer layer which is a "release coat".

### BACKGROUND OF THE INVENTION

Coated solid pharmaceutical dosage forms are commonly known in the art. Dosage forms may be coated for a variety of reasons including to protect unstable core compositions, provide a barrier coat, provide protection to the tablet in the stomach (enteric coatings), mask unpleasant taste or mouthfeel, improve general appearance, improve ease of swallowing and the like.

The use of dual or multiple coatings is also known. Multiple coatings have been used to separate unstable medicaments, separately deliver medicaments, control release, protect actives and the like.

It is known in the art to use film forming materials to coat solid oral dosage cores. A commonly used film coating is one which uses slightly water soluble film formers particularly cellulose derivatives. These are polymers which become soft by trapping water, forming viscous colloidal solutions which can be dried to form films. Hydroxypropyl methyl cellulose is most widely used and is commercially available as Opadry Brand from Colorcon. Hydroxypropyl methyl cellulose does not readily dissolve in saliva in the time in which a tablet is in the oral cavity thus making this type coating excellent for use on pharmaceutical tablets to protect an active medicament core and mask bitter taste. US 5,098,715 (incorporated by reference) teaches that coatings of this type, particularly hydroxypropyl methyl cellulose coatings, particularly Opadry brand coatings, can be used to deliver volatile flavoring/cooling agents in thin film coatings, particularly for use on unpleasant tasting core ingredients to enhance the taste-masking.

The use of gel forming mucoadhesives, such as carrageenan, in film coatings is also known. US 2005/0196445, US 2004/0137043, US 6,709,713, US 6,723,342 and US 6,432,448 (incorporated by reference) teach coating compositions based on a combination of carrageenan and microcrystalline cellulose, and, optionally, a plasticizer or a strengthening polymer or a surfactant. These coatings are taught to have excellent appearance, provide the advantages of being readily dispersed and rapidly hydrated in aqueous media allowing for ease of manufacture, and as quickly releasing active ingredients from the core. Carrageenan-based coatings of this type are commercially available from FMC Corporation as LustreClear brand coatings.

There still exists however, a need to provide a coated dosage form wherein the coating has both the strength and excellent appearance of the hydroxypropyl methylcellulose type coatings, but which can quickly release taste affecting ingredients into the oral cavity, while at the same time still mask the unpleasant taste of, and offering protection to, a medicament in the core.

### SUMMARY OF THE INVENTION

The present invention is directed to a film coated solid dosage form comprising a solid core and at least two separately applied coatings or layers comprising a first layer which is a seal coat containing a swellable polymer, esp. hydroxypropyl methyl cellulose, and a second layer which is a release coat containing (i) carrageenan (ii)microcrystalline cellulose (iii) hydroxypropyl methyl cellulose (iv) flavors, and (v) sweeteners, wherein the film coatings are separately applied to the core.

### DETAILED DESCRIPTION OF THE INVENTION

Unless otherwise indicated ingredient terms are to be understood to include one or more of the described ingredient. The term "taste modifying agent" should be understood to include flavors and sweeteners. The term "flavors" should be understood to include flavoring agents, cooling agents and the like, as described herein.

It has been discovered that prior art, carrageenan-based coatings containing microcrystalline cellulose, such as the commercially available LustreClear brand coating, can be used to deliver taste affecting ingredients such as flavors and sweeteners to the oral cavity. When flavors (volatile and non-volatile) and sweeteners are combined with a carrageenan-based coating formulation, solid dosage forms, particularly tablets, coated with the formulation provide a quick release of the flavors and sweeteners. Wherein the flavors include cooling agents, an intense cooling effect is instantly perceived by the consumer upon placing the tablet in the oral cavity.

However, when used on cores containing sensitive medicaments, the organoleptic properties of the coating are affected. The coatings become fragile, not robust, discolored, and generally unacceptable in appearance. Sensitive medicaments are those which can be thermodynamically unstable, chemically reactive, unstable to light, heat or moisture and, hence a core in which they are used can change either chemically or physically. The chemical or physical changes to the core can result in the physical or the organoleptic properties of a coating applied thereto being changed. In the present case it is believed that some of the negative results occur as a result of the interaction of the flavoring/cooling agents in the coating itself, with the active in the core.

It has been discovered that the above problem can be overcome, in part, by applying a first layer to the core prior to the application of the carrageenan-based layer. The use of a first coating, particularly of the type containing a polymer such as hydroxypropyl methyl cellulose, acts as a barrier between the unstable core composition and the carrageenan-based layer. The use of this first coating likewise prevents any interaction of the flavoring/cooling agents in a subsequently applied carrageenan-based outer layer with the core.

It was found however that the use of the two coatings per se still does not provide a robust coated product, one with desirable appearance, and does not allay all color change problems. Further, the two coatings do not achieve a desired bonding of the two layers to form an acceptable product.

It was discovered that a quick release, robust coating can be provided only when the outer coating layer is formulated in a specific manner. It has been discovered that by combining an amount of hydroxypropyl methyl cellulose, with the carrageenan and microcrystalline cellulose in the outer layer, an organoleptically acceptable coated dosage form can be provided. The resultant dual coated dosage form is found to provide excellent coating strength, surface appearance and good bonding between layers without sacrificing the property of a quick release of the taste modifying agents.

The invention, therefore, essentially provides a taste releasing, coated, solid oral dosage form having a solid core, a first layer which is a "protective seal coat" and a second outer layer which is a "release coat". The "protective seal coat" provides a barrier between the core and the outer coating. The "release coat" provides quick release of the taste modifying agents therein, while also providing good adherence to the first coating and excellent appearance and organoleptic properties to the product.

The first layer requires as the coating material a slightly soluble, swellable film former polymer such as hydroxypropyl methyl cellulose. The second layer requires hydroxypropyl methyl cellulose combined with carrageenan and microcrystalline cellulose. The second layer also contains taste modifying agents which preferably are volatile and non-volatile flavoring/cooling agents together with sweeteners, and optional colors. The dual layer coating can deliver the ready release of non-volatile flavoring and cooling agents together with volatile agents, can provide a robust texture, a shinny, smooth appearance, have no color changes, excellent mouthfeel and integrity on packaging.

The amount of hydroxypropyl methyl cellulose to be used in the second coating layer suitably.should be such that the flavor/sweetener is immediately released, but not so little as to not insure a robust texture to the outer coating layer. The hydroxypropyl methyl cellulose may range from about 1% up to 40% by weight of solids in the coating formula. A suitable formula would contain hydroxypropyl methyl cellulose in the range of from 2% to 20% by weight, preferably from 5% to 15% by weight.

The second coating formula further comprises carrageenan and microcrystalline cellulose. The amount of carrageenan in the coating formula may range from about 1% to about 6% by weight of solids in the mixture, preferably from about 2% to 5% by weight. The amount of microcrystalline cellulose may range from about 4% to about 15% by weight of solids in the mixture, preferably from 7% to 10% by weight. The ratio of microcrystalline cellulose/carrageenan to be used in the coating formula is suitably in the range of from about 65/35 to 90/10, with 70/30 to 85/15 preferred.

The second coating formula further preferably contains a plasticizer. Examples include but are not limited to polyethylene glycol; polyvinylpyrrolidone; polypropylene glycol polyvinylalcohol; polyvinyl acetate; polyhydric alcohols such as glycerol, sorbitol, mannitol, propylene glycol; esters of polyhydric alcohols such as glycerol triacetate, triacetin, glycerol tricaprylate, monacetin and diacetin and mixtures thereof and the like. The plasticizer is generally used in amounts sufficient to provide the desired degree of flexibility and toughness to the coating being applied and to facilitate processing. Although the plasticizer is optional, it is preferably used. Usually the plasticizer is present in amounts in the range of from about 2% to about 15% by weight with 3% to 8% preferred. The preferred plasticizer is polyethylene glycol.

Flavors and sweeteners are also used in the second coating layer. The flavors are selected from flavoring agents and/or cooling agents and the like. The flavoring agent may also provide the cooling effect and vice versa. One or more components may comprise each of the flavoring agent and/or cooling agent and the sweetener. Flavors may be present in an amount of up to 80% of the coating by weight. A suitable formula would contain from about 40% to 75% by weight of the coating. The sweetener may be present in an amount of from about 0.01% to 5% by weight of the coating.

The flavoring agents that can be used include those known to the skilled artisan, such as natural and artificial flavoring agents. These flavorings may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, wintergreen, eucalyptus clove oil, bay oil, thyme oil, cedar leaf oil, oil of nutmeg, oil of sage, and oil of bitter almonds. Also useful are artificial, natural or synthetic flavors such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth, flavoring agents such as eucalyptol, thymol, camphor, methyl salicylate, benzaldehyde, ginger and the like, acidulants such as citric acid, malic acid and the like. Flavorings such as aldehydes and esters including cinnamyl acetate, cinnamaldehyde, citral, diethylacetal, dihydrocarvyl acetate, eugenyl formate, p-methylanisole, and so forth may also be used. Agents which can provide a sensation of heat may also be used. These include but are not limited to capsicum, capsaicinoids, pipperine, gingerols, isothiocyanates, and materials such as chili pepper, horseradish, ginger, black pepper and the like. Generally, any flavoring or food additive, such as those described in Chemicals Used in Food Processing, publication 1274 by the National Academy of Sciences, pages 63-258, may be used. These flavorings can be used individually or in admixture.

Agents known as cooling agents include, but are not limited to, menthol, substituted p-menthanes, e.g., hydroxymethyl and hydroxyethyl derivatives, menthyl succinate (PHYSCOOL), menthyl lactates (e.g. FRESCOLAT), N,N-dimethyl menthyl succinamide; substituted-p-menthane-3-carboxamides, such as N-ethyl-p-menthane-3-carboximide (WS-3), N,2,3-trimethyl-2-isopropyl butamide (WS-23); acyclic carboxamides, substituted cyclohexanamides, substituted cyclohexane carboxamides, substituted ureas and sulphonamides, and substituted menthanols; 3-1-menthoxy propan-1,2-diol, menthoxypropane diol, menthone glycerol ketals, p-menthane-3,8-diols, 2-mercapto-cyclo-decanone, 2-isopropanyl-5-methylcyclohexanol (ISOPREGOL); isopulegol, cubebol, incilin, xylitol and others compounds known for their cooling effects and mixtures thereof.

A preferred embodiment contains a combination of a flavoring agent and a cooling agent.

The sweetener may be a sugar or may be a sugar substitute or mixtures thereof. Useful sweeteners include, but are not limited to sugars such as sucrose, glucose (corn syrup), dextrose, invert sugar, fructose, and mixtures thereof; saccharin and its various salts such as the sodium or calcium salt; cyclamic acid and its various salts such as the sodium salt; the dipeptide sweeteners such as aspartame, alitame, neotame; sucralose, natural sweeteners such as dihydrochalcone compounds; glycyrrhizin; Stevia rebaudiana (Stevioside); sugar alcohols such as sorbitol, sorbitol syrup, mannitol, xylitol and the like, synthetic sweeteners such as acesulfame-K and sodium and calcium salts thereof and the like, hydrogenated starch hydrolysate (lycasin); protein based sweetening agents such as talin (thaumaoccous danielli) and/or any other pharmacologically acceptable sweetener known by the state of the art, and mixtures thereof.

The sweetener is preferably a high intensity sweetener with sucralose being especially preferred.

A color or opacifying agent is optional but preferred. Where color is used, the two layers can have the same or different colors. The amount of color or opacifying agent in each layer can vary depending on the desired effect and can range from about 3% to 30% by weight.

The coloring or opacifying agents useful in the present invention include pigments such as titanium dioxide and natural food colors and dyes suitable for food, drug and cosmetic applications. These colorants are known as FD&C dyes and lakes. A full recitation of all FD&C and D&C dyes and their corresponding chemical structures may be found in the Kirk-Othmer Encyclopedia of Chemical Technology, Volume 5, Pages 857-884, which text is accordingly incorporated herein by reference.

A formula for the second coating layer would suitably contain the following components and amounts:

**Table 1: Outer Coating Formula**

| **Ingredient** | **% By Dry Weight** |
|---|---|
| Carrageenan | 1 - 6 |
| Microcrystalline Cellulose | 4-15 |
| Hydroxypropyl Methyl Cellulose | 1 - 40 |
| Flavors | 20 - 80 |
| Sweeteners | 0.01 - 5 |
| Plasticizer | 2 - 15 |
| Color or Opacifying Agent | 3 - 30 |

Commercially available film coating formers such as LustreClear Brand of FMC can be used in the outer coating. LustreClear will generally contain carrageenan in the range of about 5% to 25% by weight with microcrystalline cellulose present in a range of from about 15% to 60 % by weight. Other components such as plasticizers may be present in ranges of from 10% to 40% by weight. LustreClear 103 for example is a commercially available combination of carrageenan, microcrystalline cellulose and polyethylene glycol. Other LustreClear formulations may contain carrageenan, microcrystalline cellulose, polyethylene glycol, hydroxyethyl cellulose and maltodextrin.

The first coating layer, or "protective coat" is comprised of slightly water soluble film formers. Suitable pharmaceutically acceptable film formers include but are not limited to cellulose based polymers such as hydroxyalkyl celluloses, for example, hydroxypropy cellulose or hydroxypropyl methyl cellulose; the alkylcelluloses, for example, methylcellulose, ethylcellulose or propylcellulose. Preferred is hydroxypropyl methyl cellulose. The film former may comprise 100% of the layer. It may be present in amounts of from 40% to 95% by weight when used with optional components.

The first layer formula may optionally contain a plasticizer such as those described above. The plasticizer may be present in amounts from about 2% to about 30% by weight of the coating. The first coating may also contain a colorant or opacifying agent as described above. The colorant or opacifying agent may be present in an amount of from about 3% to about 30% by weight.

The first coating layer may suitably be comprised of a commercial film-coating product designed for aqueous film coating containing the polymer hydroxypropyl methyl cellulose. Such products are commercially available under the trade name Opadry from Colorcon, West Point, Pa. A suitable blend comprises about 40% to about 95% by weight hydroxypropyl methyl cellulose, optionally 3% to about 20% by weight colorant or opacifying agent, and optionally from 4 to about 25% by weight plasticizer.

The Opadry used as the first coating layer, may also be used as a source of hydroxypropyl methyl cellulose to combine with the LustreClear to provide the second, release layer. The coating combining LustreClear and Opadry would contain the LustreClear/Opadry in ratios from about 5/1 to 1/2 by weight. A most preferred ratio would be from 4/1 to 1/1 by weight.

The cores used in the practice of the invention may be a placebo or any suitable solid dosage cores containing an active substance. The compositions according to the present invention may take the form of tablets, pellets, pills, powders, granules or capsules for oral administration. These forms are distinguished from soft dosage forms such as gelatin capsules, hard gums and the like. Preferred cores are tablets. The composition and manufacture of tablet cores are well known in the art.

In the preferred embodiment the core contains an effective amount of a medicament. The invention is particularly useful for application to sensitive medicaments, that is, medicaments which can react with certain materials with which they contact. However the medicament can be any active substance deliverable by a solid dosage form and can be selected from classes from those in the following therapeutic categories whether or not the medicaments are found to be sensitive: ace-inhibitors; alkaloids; antacids; analgesics; anabolic agents; anti-anginal drugs; anti-allergy agents; anti-arrhythmia agents; antiasthmatics; antibiotics; anticholesterolemics; anticonvulsants; anticoagulants; antidepressants; antidiarrheal preparations; anti-emetics; antihistamines; antihypertensives; anti-infectives; antiinflammatories; antilipid agents; antimanics; anti-migraine agents; antinauseants; antipsychotics; antistroke agents; antithyroid preparations; anabolic drugs; antiobesity agents; antiparasitics; antipsychotics; antipyretics; antispasmodics; antithrombotics; antitumor agents; antitussives; anti-ulcer agents; anti-uricemic agents; anxiolytic agents; appetite stimulants; appetite suppressants; beta-blocking agents; bronchodilators; cardiovascular agents; cerebral dilators; chelating agents; cholecystekinin antagonists; chemotherapeutic agents; cognition activators; contraceptives; coronary dilators; cough suppressants; decongestants; diabetes agents; diuretics; enzymes; erythropoietic drugs; expectorants; fertility agents; gastrointestinal agents; growth regulators; hormone replacement agents; hyperglycemic agents; hypoglycemic agents; laxatives; migraine treatments; mineral supplements; mucolytics, narcotics; neuroleptics; neuromuscular drugs; non-steroidal anti-inflammatory drugs (NSAIDs); nutritional additives; peripheral vasodilators; polypeptides; prostaglandins; psychotropics; renin inhibitors; respiratory stimulants; sedatives; steroids; stimulants; sympatholytics; thyroid preparations; tranquilizers; uterine relaxants; vasoconstrictors; vasodilators; vertigo agents; vitamins; and the like.

Active agents that may be used in the invention include, but are not limited to: acetaminophen; acetylsalicylic acid, including its buffered forms; acrivastine; albuterol and its sulfate; alkaline phosphatase; allantoin; aluminum acetate, carbonate, chlorohydrate and hydroxide; alprozolam; amino acids; aminobenzoic acid; amoxicillin; ampicillin; amsacrine; amsalog; anethole; ascorbic acid; astemizole; atenolol; azatidine and its maleate; bacitracin; BCNU (carmustine); beclomethasone diproprionate; benzophenones; benzquinamide and its hydrochloride; bethanechol; biotin; bisacodyl; bismuth subsalicylate; bornyl acetate; bromopheniramine and its maleate; buspirone; caffeine; calcium carbonate, casinate and hydroxide; camphor; captopril; cascara sagrada; cefaclor; cefadroxil; cephalexin; centrizine, cetirizine; cetylpyridinium chloride; chloramphenicol; chlorcyclizine hydrochloride; chlorhexidine gluconate; chloroxylenol; chloropentostatin; chlorpheniramine and its maleates and tannates; chlorpromazine; cholestyramine resin; choline bitartrate; chondrogenic stimulating protein; cimetidine; cinnamedrine hydrochloride; citalopram; clarithromycin; clemastine and its fumarate; clonidine; clorfibrate; codeine and its fumarate and phosphate; cortisone acetate; ciprofloxacin HCl; cyanocobalamin; cyclizine hydrochloride; cyproheptadine; danthron; dexbromopheniramine maleate; dextromethorphan and its hydrohalides; diazepam; dibucaine; dichloralphenazone; diclofen and its alkali metal sales; diclofenac sodium; digoxin; dihydroergotamine and its hydrogenates/mesylates; diltiazem; dimethicone; dioxybenzone; diphenhydramine and its citrate; diphenhydramine and its hydrochloride; divalproex and its alkali metal salts; docusate calcium, potassium, and sodium; doxycycline hydrate; doxylamine succinate; efaroxan; enalapril; enoxacin; ergotamine and its tartrate; erythromycin; estropipate; ethinyl estradiol; ephedrine; epinephrine bitartrate; erythropoietin; eucalyptol; famotidine; fenoprofen and its metal salts; ferrous fumarate, gluconate and sulfate; fexofenadine; fluoxetine; folic acid; fosphenytoin; 5-fluorouracil (5-FU); fluoxetine; flurbiprofen; furosemide; gabapentin; gentamicin; gemfibrozil; glipizide; glycerine; glyceryl stearate; granisetron; griseofulvin; growth hormone; guafenesin; hexylresorcinol; hydrochlorothiazide; hydrocodone and its tartrates; hydrocortisone and its acetate; 8-hydroxyquinoline sulfate; hydroxyzine and its pamoate and hydrochloride salts; ibuprofen; indomethacin; inositol; iron; isosorbide and its mono- and dinitrates; isoxicam; ketamine; ketoprofen; lecithin; leuprolide acetate; lidocaine and its hydrochloride salt; lifinopril; liotrix; loperamide, loratadine; lovastatin; luteinizing hormore; LHRH (lutenizing hormone replacement hormone); magnesium carbonate, hydroxide, salicylate, and trisilicate; meclizine; mefenamic acid; meclofenamic acid; meclofenamate sodium; medroxyprogesterone acetate; methenamine mandelate; meperidine hydrochloride; metaproterenol sulfate; methscopolamine and its nitrates; methsergide and its maleate; methyl nicotinate; methyl salicylate; methsuximide; metoclopramide and its halides/hydrates; metronidazole; metoprotol tartrate; miconazole nitrate; minoxidil; morphine; naproxen and its alkali metal sodium salts; nifedipine; neomycin sulfate; niacin; niacinamide; nicotine; nicotinamide; nimesulide; nitroglycerine; nonoxynol-9; norethindrone and its acetate; nystatin; omega-3 polyunsaturated fatty acids; omeprazole; ondansetron and its hydrochloride; oxolinic acid; oxybenzone; oxtriphylline; padimate-O; paramethadione; pentastatin; pentaerythritol tetranitrate; pentobarbital sodium; perphenazine; phenelzine sulfate; phenindamine and its tartrate; pheniramine maleate; phenobarbital; phenolphthalein; phenylephrine and its tannates and hydrochlorides; phenylpropanolamine; phenytoin; pirmenol; piroxicam and its salts; polymicin B sulfate; potassium chloride and nitrate; prazepam; procainamide hydrochloride; procaterol; promethazine and its hydrochloride; propoxyphene and its hydrochloride and napsylate; pramiracetin; pramoxine and its hydrochloride salt; prochlorperazine and its maleate; propanolol and its hydrochloride; promethazine and its hydrochloride; propanolol; pseudoephedrine and its sulfates and hydrochlorides; pyridoxine; pyrolamine and its hydrochlorides and tannates; quinapril; quinidine gluconate and sulfate; quinestrol; ralitoline; ranitadine; resorcinol; riboflavin; salicylic acid; scopolamine; sodium bicarbonate, citrate, and fluoride; sodium monofluorophosphate; sucralfate; sulfanethoxazole; sulfasalazine; sulfur; sumatriptan and its succinate; tacrine and its hydrochloride; theophylline; terfenadine; thiethylperazine and its maleate; timolol and its maleate; thioperidone; tramadol; trimetrexate; triazolam; tretinoin; tetracycline hydrochloride; tolmetin; tolnaftate; trimethobenzamide and its hydrochloride; tripelennamine and its hydrochloride; tripolidine hydrochloride; undecylenic acid; vancomycin; verapamil HCl; vidaribine phosphate; vitamins A, B.sub.1-12, C, D, E, and K; xylometazoline hydrochloride; zinc. Any of the active agents set forth above, pharmaceutically acceptable salts thereof, pharmaceutically acceptable enantiomers thereof; and mixtures thereof are also suitable for use in the present invention.

In an embodiment the invention is preferably used for those active substances which may react with a coating applied to the core containing the active. These include but are not limited to ranitidine, phenylephrine, dimenhydrinate, cetirizine, pseudoephedrine, guafenesin, acetaminophen, acetylsalicylic acid, ibuprofen, chlorpheniramine, nicotine and naproxen.

The dual layers are separately applied to the cores and maintain as separate layers. The core can be panned or spray coated with each layer to provide a uniform surface or finish. Other conventional methods of forming the core are within the scope of the invention. For example, an acceptable coating application system includes without limitation, a plain fluid bed system, including a fluid bed spray tower. Also acceptable to prepare the coated core of this invention are a variety of side vented coating pans, spray dryers, continuous coating pans, and conventional coating pans.

The preferred method for applying each coating comprises standard aqueous spray-coating techniques wherein core tablets are continuously spray-coated with the film coating solution. The process comprises the step of first admixing the components for each layer until hydration is complete, approximately 2 hrs. The formulated core tablets are placed in a coating chamber; the coating material is sprayed into the coating chamber until the coated tablets show a desired weight increase. The preferred method of the invention comprises a one-step continuous spray-coating process to apply the film coating.

The film coating per se may range from about 4% to about 8% by weight of product. The preferred coating would be from 5% to 7%. Each coating layer may range from about 2% to about 4% by weight of the product.

While certain preferred and alternative embodiments of the invention have been set forth for purposes of disclosing the invention, modification to the disclosed embodiments can occur to those who are skilled in the art. The following examples serve to provide further appreciation of the invention but are not meant in any way to restrict the effective scope of the invention.

### EXAMPLES:

### Example 1: Ranitidine Tablets

Components were prepared as follows.

**Table 2: Tablet Cores**

| **Ingredient** | **% Weight Dry** | **Mg Per Piece** |
|---|---|---|
| Ranitidine hydrochloride | 56.0000 | 168.0000 |
| Microcrystalline cellulose | 43.2500 | 129.7500 |
| Magnesium stearate | 0.7500 | 2.2500 |
| **Total** | 100.00 | 300.0000 |

The ingredients were blended and compressed to form 300 mg tablets.

**Table 3: First Coat:**

| **Ingredient** | **% Dry Weight** | **Mg, Wet Weight** |
|---|---|---|
| Opadry Blue* | 100.00 | 7.5000 |
| Water | --- | 67.5000 |
| **Total** | 100.00 | 75.0000 |

| | | |
|---|---|---|
| *blend of hydroxypropyl methyl cellulose, colorant and polyethylene glycol | | |

The Opadry was added to the water with blending until hydrated.

**Table 4: Second Coat:**

| **Ingredient** | **% Dry Weight** | **Mg, Wet Weight** |
|---|---|---|
| LustreClear LC103** | 22:8576 | 1.7143 |
| Wintergreen flavor | 44.6606 | 3.3495 |
| Peppermint flavor | 15.5509 | 1.1663 |
| Sucralose | 0.3107 | 0.0233 |
| WS-23 (trimethyl-isopropylbutamide) | 3.0067 | 0.2255 |
| Opadry Blue* | 13.6135 | 1.0210 |
| Water | --- | 26.0333 |
| **Total** | 100.00 | 34.0333 |

| | | |
|---|---|---|
| *blend of hydroxypropyl methyl cellulose, colorant and polyethylene glycol **blend of microcrystalline cellulose, carrageenan, polyethylene glyol | | |

The Opadry was added to an amount of the water to form a 10 %, solution and blended until hydrated. The peppermint flavor, the remaining water and the LustreClear were added to a separate container. The wintergreen flavor, sucralose and WS-23 were separately pre-mixed then added to the LustreClear mix. The combination was blended until hydrated (approximately 2 hrs). The separately hydrated Opadry was then added to the LustreClear blend and the combination further blended for approximately 15 minutes to prepare the coating solution.

**Table 5: Coated Tablet:**

| **Component** | **% Dry Weight** | **Mg per Piece** |
|---|---|---|
| Ranitidine core | 95.2381 | 300.0000 |
| First coating solution | 2.3810 | 7.5000 |
| Second coating solution | 2.3809 | 7.4999 |
| **Total** | 100.00 | 314.9999 |

Each coating was applied to the tablet core using an ACCELACOTA 24" pan sprayer rotating at 12rpm with an outlet temperature of 39°C and a spray rate of 20gms/gun/minute. The gun placement: was approximately 8 inches from the tablet bed. Each coating was applied to achieve the desired weight.

The tablets were found to have desirable organoleptic qualities with excellent adherence of the coating layers.

### Example 2: Placebo

The placebo cores were prepared as follows:

**Table 6: Tablet Cores**

| **Ingredient** | **% Dry Weight** | **Mg per Piece** |
|---|---|---|
| Microcrystalline cellulose | 99.2500 | 297.7500 |
| Magnesium stearate | 0.7500 | 2.2500 |
| **Total** | 100.00 | 300.0000 |

The first and second coat layers were formulated as in Example 1, Tables 3 and 4 and the coated tablets as in Table 5. The coated tablets were prepared in a manner similar to those prepared in Example 1.

The coated tablets were evaluated in a consumer taste test (282 subjects) for acceptability of the coating's taste and cooling sensation. The consumers overwhelmingly evaluated the product as having a cooling sensation with an immediate cooling effect that was felt in the mouth and throat and remained as a pleasing aftertaste.

### Example 3: Phenylephrine Tablets

Components are formulated as follows:

**Table 7: Tablet Cores**

| **Ingredient** | **% Dry Weight** | **Mg per Piece** |
|---|---|---|
| Phenylephrine HCl | 6.76 | 10.00 |
| Silicafied Microcrystalline cellulose | 80.81 | 119.60 |
| Pregelatinized starch | 2.70 | 4.00 |
| Crospovidone | 4.05 | 6.00 |
| Stearic acid | 5.41 | 8.00 |
| Magnesium stearate | 0.27 | 0.400 |
| **Total** | 100.00 | 148.00 |

**Table 8: First Coat**

| **Ingredients** | **% Dry Weight** | **Mg, Wet Weight** |
|---|---|---|
| Opadry Red* | 100 | 3.70 |
| Water | --- | 33.30 |
| Total | 100 | 37.00 |

| | | |
|---|---|---|
| *blend of hydroxypropyl methyl cellulose, colorant and polyethylene glycol | | |

**Table 9: Second Coat**

| **Ingredients** | **% Dry Weight** | **Mg, Wet Weight** |
|---|---|---|
| LustreClear 103** | 22.8576 | 0.846 |
| Wintergreen | 44.6606 | 1.65 |
| Peppermint | 15.5509 | 0.5750 |
| Sucralose | 0.3107 | 0.0115 |
| WS-23 | 3.0067 | 0.1112 |
| Opadry Red* | 13.6135 | 0.5037 |
| Water | --- | 2.81 |
| Total | 100 | 3.70 |

| | | |
|---|---|---|
| *blend of hydroxypropyl methyl cellulose, colorant and polyethylene glycol **blend of microcrystalline cellulose, carrageenan, polyethylene glyol | | |

The components are prepared similar to Example 1. The coated tablet is formulated as follows.

**Table 10: Coated Tablet:**

| **Component** | **% Dry Weight** | **Mg per Piece** |
|---|---|---|
| Phenylephrine Tablet Core | 95.2381 | 300.0000 |
| First coating solution | 2.3810 | 7.5000 |
| Second coating solution | 2.3809 | 7.4999 |
| **Total** | 100.00 | 314.9999 |

The coated tablet is prepared in a manner similar to Example 1

### Example 4: Phenylephrine/Acetaminophen Tablets

Components are formulated as follows:

**Table 11: Tablet Cores**

| **Ingredient** | **% Dry Weight** | **Mg Per Piece** |
|---|---|---|
| Phenylephrine HCl | 1.0781 | 5.00 |
| Acetaminophen USP | 77.859 | 361.11 |
| Silicafied Microcrystalline cellulose | 12-2661 | 56.89 |
| Pregelatinized starch | 1.9405 | 9.00 |
| Crospovidone | 1.9405 | 9.00 |
| Stearic acid | 1.9405 | 9.00 |
| **Total** | 100.00 | 450.00 |

**Table 12: First Coat**

| **Ingredient** | **% Dry Weight Mg,** | **Wet Weight** |
|---|---|---|
| Opadry Orange* | 10 | 1.1.25 |
| Water | 90 | 101.25 |
| Total | 100 | 112.50 |

| | | |
|---|---|---|
| *blend of hydroxypropyl methyl cellulose, colorant and polyethylene glycol | | |

**Table 13: Second Coat**

| **Ingredients** | **% Dry Weight** | **Mg, Wet Weight** |
|---|---|---|
| LustreClear 103** | 22.8576 | 2.570 |
| Wintergreen | 44.6606 | 5.0250 |
| Peppermint | 15.5509 | 1.750 |
| Sucralose | 0.3107 | 0.035 |
| WS-23 | 3.0067 | 0.338 |
| Opadry Red* | 13.6135 | 1.532 |
| Water( lost on drying) | --- | 8.606 |
| Total dry weight gain | 100 | 19.856 |

| | | |
|---|---|---|
| *blend of hydroxypropyl methyl cellulose, colorant and polyethylene glycol **blend of microcrystalline cellulose, carrageenan, polyethylene glyol | | |

The components are prepared similar to Example 1. The coated tablet is formulated as follows.

**Table 14: Coated Tablet:**

| **Component** | **% Dry Weight** | **Mg Per Piece** |
|---|---|---|
| Phenylephrine/Acetaminophen Tablet Core | 95.2381 | 300.0000 |
| First coating solution | 2.3810 | 7.5000 |
| Second coating solution | 2.3809 | 7.4999 |
| **Total** | 100.00 | 314.9999 |

The coated tablet is prepared in a manner similar to Example 1

## Claims

1. A film-coated solid oral dosage form comprising
a) a solid core, and
b) a film coating comprising at least two separately applied layers comprising
1) a first layer comprising a swellable polymer
2) a second layer comprising
(i) carrageenan
(ii) microcrystalline cellulose
(iii) hydroxypropyl methyl cellulose
(iv) flavor
(v) sweetener
wherein said film coatings are applied to said core.

2. The dosage form of claim 1 wherein said swellable polymer in said first layer is hydroxypropyl methyl cellulose.

3. The dosage form of claim 2 wherein said hydroxypropyl methyl cellulose is present in an amount of from about 40% to about 95% by weight of said layer.

4. The dosage form of claim 1 wherein said carrageenan in said second layer is present in an amount of from about 1% to about 6% by weight of said layer.

5. The dosage form of claim 1 wherein said microcrystalline cellulose in said second layer is present in an amount of from about 4% to about 15% by weight of said layer.

6. The dosage form of claim 1 wherein said hydroxypropyl methyl cellulose in said second layer is present in an amount of from about 1% to about 40% by weight of said layer.

7. The dosage form of claim 1 wherein said flavor in said second layer is present in an amount of from about 20% to about 80% by weight of said layer.

8. The dosage form of claim 1 wherein said sweetener in said second layer is present in an amount of from about 0.01% to about 5% by weight of said layer.

9. The dosage form of claim 1 wherein said solid core further comprises an active substance

10. The dosage form of claim 1 wherein said first layer further comprises a plasticizer.

11. The dosage form of claim 1 wherein said second layer further comprises a plasticizer.

12. The dosage form of claim 1 wherein said first layer further comprises a colorant or opacifying agent.

13. The dosage form of claim 1 wherein said second layer further comprises a colorant or opacifying agent.

14. The dosage form of claim 1 wherein said first layer is from about 2% to about 4% by weight of said form.

15. The dosage form of claim 1 wherein said second layer is from about 2% to about 4% by weight of said form.

16. The dosage form of claim 1 wherein said film coating is from about 4% to about 8% by weight of said form.

## Patentansprüche

1. Feste orale Dosierform mit Filmüberzug, umfassend
a) einen festen Kern und
b) einen Filmüberzug, der wenigstens zwei getrennt aufgebrachte Schichten umfasst, umfassend
1) eine erste Schicht, die ein quellbares Polymer umfasst,
2) eine zweite Schicht, umfassend
(i) Carrageenan
(ii) mikrokristalline Cellulose
(iii) Hydroxypropylmethylcellulose
(iv) Geschmacksstoff
(v) Süßungsmittel
wobei die Filmüberzüge auf den Kern aufgebracht sind.

2. Dosierform nach Anspruch 1, wobei das quellbare Polymer in der ersten Schicht Hydroxypropylmethylcellulose ist.

3. Dosierform nach Anspruch 2, wobei die Hydroxypropylmethylcellulose in einer Menge von etwa 40 bis etwa 95 Gew.-% der Schicht vorliegt.

4. Dosierform nach Anspruch 1, wobei das Carrageenan in der zweiten Schicht in einer Menge von etwa 1 bis etwa 6 Gew.-% der Schicht vorliegt.

5. Dosierform nach Anspruch 1, wobei die mikrokristalline Cellulose in der zweiten Schicht in einer Menge von etwa 4 bis etwa 15 Gew.-% der Schicht vorliegt.

6. Dosierform nach Anspruch 1, wobei die Hydroxypropylmethylcellulose in der zweiten Schicht in einer Menge von etwa 1 bis etwa 40 Gew.-% der Schicht vorliegt.

7. Dosierform nach Anspruch 1, wobei der Geschmacksstoff in der zweiten Schicht in einer Menge von etwa 20 bis etwa 80 Gew.-% der Schicht vorliegt.

8. Dosierform nach Anspruch 1, wobei das Süßungsmittel in der zweiten Schicht in einer Menge von etwa 0,01 bis etwa 5 Gew.-% der Schicht vorliegt.

9. Dosierform nach Anspruch 1, wobei der feste Kern weiter eine aktive Substanz umfasst.

10. Dosierform nach Anspruch 1, wobei die erste Schicht weiter einen Weichmacher umfasst.

11. Dosierform nach Anspruch 1, wobei die zweite Schicht weiter einen Weichmacher umfasst.

12. Dosierform nach Anspruch 1, wobei die erste Schicht weiter ein Färbemittel oder Deckmittel umfasst.

13. Dosierform nach Anspruch 1, wobei die zweite Schicht weiter ein Färbemittel oder Deckmittel umfasst.

14. Dosierform nach Anspruch 1, wobei die erste Schicht von etwa 2 bis etwa 4 Gew.-% der Form ausmacht.

15. Dosierform nach Anspruch 1, wobei die zweite Schicht von etwa 2 bis etwa 4 Gew.-% der Form ausmacht.

16. Dosierform nach Anspruch 1, wobei der Filmüberzug von etwa 4 bis etwa 8 Gew.-% der Form ausmacht.

## Revendications

1. Forme pharmaceutique orale solide enrobée d'un film comprenant
a) un noyau solide, et
b) un enrobage de film comprenant au moins deux couches appliquées séparément comprenant
1) une première couche comprenant un polymère pouvant gonfler
2) une seconde couche comprenant
(i) de la carraghénine
(ii) de la cellulose microcristalline
(iii) de l'hydroxypropylméthylcellulose
(iv) un arôme
(v) un édulcorant
dans laquelle lesdits enrobages de film sont appliqués sur ledit noyau.

2. Forme pharmaceutique selon la revendication 1, dans laquelle ledit polymère pouvant gonfler dans ladite première couche est l'hydroxypropylméthylcellulose.

3. Forme pharmaceutique selon la revendication 2, dans laquelle ladite hydroxypropylméthylcellulose est présente en une quantité comprise dans la plage allant d'environ 40 % à environ 95 % en poids de ladite couche.

4. Forme pharmaceutique selon la revendication 1, dans laquelle ladite carraghénine dans ladite seconde couche est présente en une quantité comprise dans la plage allant d'environ 1 % à environ 6 % en poids de ladite couche.

5. Forme pharmaceutique selon la revendication 1, dans laquelle ladite cellulose microcristalline dans ladite seconde couche est présente en une quantité comprise dans la plage allant d'environ 4 % à environ 15 % en poids de ladite couche.

6. Forme pharmaceutique selon la revendication 1, dans laquelle ladite hydroxypropylméthylcellulose dans ladite seconde couche est présente en une quantité comprise dans la plage allant d'environ 1 % à environ 40 % en poids de ladite couche.

7. Forme pharmaceutique selon la revendication 1, dans laquelle ledit arôme dans ladite seconde couche est présent en une quantité comprise dans la plage allant d'environ 20 % à environ 80 % en poids de ladite couche.

8. Forme pharmaceutique selon la revendication 1, dans laquelle ledit édulcorant dans ladite seconde couche est présent en une quantité comprise dans la plage allant d'environ 0,01 % à environ 5 % en poids de ladite couche.

9. Forme pharmaceutique selon la revendication 1, dans laquelle ledit noyau solide comprend en outre une substance active.

10. Forme pharmaceutique selon la revendication 1, dans laquelle ladite première couche comprend en outre un plastifiant.

11. Forme pharmaceutique selon la revendication 1, dans laquelle ladite seconde couche comprend en outre un plastifiant.

12. Forme pharmaceutique selon la revendication 1, dans laquelle ladite première couche comprend en outre un colorant ou un agent opacifiant.

13. Forme pharmaceutique selon la revendication 1, dans laquelle ladite seconde couche comprend en outre un colorant ou un agent opacifiant.

14. Forme pharmaceutique selon la revendication 1, dans laquelle ladite première couche représente d'environ 2 % à environ 4 % en poids de ladite forme pharmaceutique.

15. Forme pharmaceutique selon la revendication 1, dans laquelle ladite seconde couche représente d'environ 2 % à environ 4 % en poids de ladite forme pharmaceutique.

16. Forme pharmaceutique selon la revendication 1, dans laquelle ledit enrobage de film représente d'environ 4 % à environ 8 % en poids de ladite forme pharmaceutique.
